(19) Europäisches Patentamt
European Patent Office
Office européen des brevets

(11) **EP 3 368 880 B1**

(12) **EUROPÄISCHE PATENTSCHRIFT**

(45) Veröffentlichungstag und Bekanntmachung des Hinweises auf die Patenterteilung:
**30.09.2020 Patentblatt 2020/40**

(51) Int Cl.:
*G01N 11/08* (2006.01)     *G01N 33/44* (2006.01)

(21) Anmeldenummer: **16759691.5**

(86) Internationale Anmeldenummer:
**PCT/EP2016/069370**

(22) Anmeldetag: **16.08.2016**

(87) Internationale Veröffentlichungsnummer:
**WO 2017/071853 (04.05.2017 Gazette 2017/18)**

(54) **VORRICHTUNG UND VERFAHREN ZUR VISKOSITÄTSBESTIMMUNG**

DEVICE AND METHOD FOR DETERMINING VISCOSITY

DISPOSITIF ET PROCÉDÉ DE DÉTERMINATION DE LA VISCOSITÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **27.10.2015 DE 102015220966**

(43) Veröffentlichungstag der Anmeldung:
**05.09.2018 Patentblatt 2018/36**

(73) Patentinhaber: **Bayerische Motoren Werke Aktiengesellschaft**
**80809 München (DE)**

(72) Erfinder: **ESCHL, Johannes**
**82194 Gröbenzell (DE)**

(56) Entgegenhaltungen:
EP-A1- 2 439 045     CN-A- 101 008 601
US-A- 4 425 790     US-A- 4 876 882
US-A- 5 076 096     US-A- 5 253 981

**EP 3 368 880 B1**

**Beschreibung**

Die vorliegende Erfindung betrifft eine Vorrichtung zur Viskositätsbestimmung von Reaktionsharzen sowie ein Verfahren zur Herstellung einer solchen Vorrichtung. Darüber hinaus betrifft die Erfindung auch ein einfach durchführbares Verfahren zur Viskositätsbestimmung.

Viskositätsbestimmungen werden zur Beurteilung des Fließverhaltens von Substanzen und Reaktionsgemischen angewandt. Unterschiedliche Messverfahren sind hierzu gebräuchlich. Bei den Messverfahren unterscheidet man solche, die eine Kraft (z.B. ein Drehmoment) bei einer bestimmten Schergeschwindigkeit messen und solche, die die Viskosität aus der Zeit bestimmen, die eine zu untersuchende Probe benötigt, um durch eine Kapillare mit definiertem Volumen zu fließen. Das erst genannte Messverfahren ist zur Viskositätsbestimmung von Reaktionsharzen ungeeignet, da die Probenvorbereitung zu lange dauert und die Harze bei Durchführung der eigentlichen Messung bereits im Aushärten begriffen sind, was zu einer Verfälschung der Messergebnisse führt. Nachteilig am zweiten genannten Messverfahren ist, dass die Dosierung von reagierenden Substanzen, sowie deren Temperierung schwierig und aufwendig ist. Ebenso ist eine Wiederverwertung der Kapillare bei Vermessung von Reaktionsharzen in der Regel nicht gegeben.

US 5076096 offenbart, zum Beispiel, ein Verfahren zur Bestimmung der Viskosität einer wärmehärtenden Zusammensetzung, wobei die Zusammensetzung durch eine Kapillare strömt und wobei der Volumenstrom und die Druckdifferenz gemessen werden.

Ausgehend von diesem Stand der Technik ist es eine Aufgabe der vorliegenden Erfindung eine Vorrichtung zur Viskositätsbestimmung von Reaktionsharzen bereitzustellen, die eine präzise Dosierung der zu reagierenden Substanzen, eine schnelle Temperierung sowie eine Viskositätsbestimmung mit geringem Fehlerpotential erlaubt und eine Aussage über das zeitliche Verarbeitungsfenster von Reaktionsharzen ermöglicht. Darüber hinaus ist es eine Aufgabe der vorliegenden Erfindung ein Verfahren zur Herstellung einer Vorrichtung zur Viskositätsbestimmung von Reaktionsharzen anzugeben, das einfach umsetzbar ist und auf marktübliche Komponenten zurückgreift. Eine weitere Aufgabe ist es auch, ein Verfahren zur Viskositätsbestimmung von Reaktionsharzen anzugeben, mit dem ohne hohen Probenvorbereitungsaufwand die Viskosität von Reaktionsharzen präzise detektiert werden kann.

Die Aufgabe wird durch eine Vorrichtung nach Anspruch 1 zur Viskositätsbestimmung von Reaktionsharzen gelöst. Die einzelnen Komponenten der erfindungsgemäßen Vorrichtung werden nachfolgend beschrieben. So umfasst die Vorrichtung i) eine Probenaufbewahrungseinheit mit mindestens zwei Vorratsbehältnissen und jeweils einer Dosiereinheit. Als Vorratsbehältnis kommt jegliches Behältnis in Frage, das inert gegenüber der zu bevorratenden Substanz ist. Die Dosiereinheit erlaubt dabei ein präzises Vereinzeln und Fördern einer definierten Menge an Substanz.

Da es sich bei der erfindungsgemäßen Vorrichtung zur Viskositätsbestimmung um eine Vorrichtung zur Bestimmung der Viskosität von Reaktionsharzen handelt, sind mindestens zwei Vorratsbehältnisse vorgesehen, ein erstes zur Aufbewahrung eines härtbaren Harzes und ein zweites zur Aufbewahrung eines Härters. Harz und Härter sind im Einzelnen nicht beschränkt. Beispielhaft seien duroplastische Harze genannt, z.B. Epoxidharze oder Polyurethanharze, wobei als Härter Amine, Isocyanate und dergleichen in Frage kommen. Weitere Vorratsbehältnisse können vorgesehen sein, beispielsweise zur Aufbewahrung, Förderung und Dosierung von Reaktionsadditiven, Lösungsmitteln, Trennmitteln und dergleichen.

Ferner umfasst die erfindungsgemäße Vorrichtung mindestens eine Mischvorrichtung. Die Mischvorrichtung ist ausgelegt, um zumindest das härtbare Harz und den Härter miteinander zu vermischen. Die Mischvorrichtung kann dabei in eine Dosiervorrichtung integriert sein.

Um eine ausreichende Strömung der Reaktionsmischung des Reaktionsharzes zu erzielen, umfasst die Vorrichtung zudem eine Hochdruckpumpe. Durch die Hochdruckpumpe wird das Reaktionsgemisch, also die Mischung aus härtbarem Harz und Härter, auf einen bestimmten Druck gebracht, wodurch sich ein vordefinierter Volumenstrom einstellen lässt.

Die Hochdruckpumpe erzeugt in der Mischung aus härtbarem Harz und Härter vorteilhaft einen Maximaldruck von 100 bis 130 bar. Dies gewährleistet eine Dichtheit des Systems, die mit zunehmend höheren Drücken sinkt, so dass Reaktionsgemischanteile z.B. zwischen einem Kolben und einem Zylinder der Hochdruckpumpe ausströmen können, wodurch die Hochdruckpumpe Volumenstrom irreversibel geschädigt wird.

Durch eine Temperiereinheit wird das Reaktionsgemisch zudem temperiert. Ein Temperieren kann sowohl ein Erhöhen der Temperatur des Reaktionsgemisches als auch ein Erniedrigen seiner Temperatur, wie es z.B. während der Härtungsreaktion üblich ist, umfassen.

Die Viskosität wird anschließend beim Durchströmen des Reaktionsgemisches durch vier oder fünf in Reihe geschaltete Kapillaren ermittelt. Eine Kapillare umfasst dabei einen Einlass, in den das Reaktionsgemisch eingelassen wird, und einen Auslass, durch den das Reaktionsgemisch die Kapillare nach Durchströmen derselben verlässt. Mindestens an jedem Auslass einer Kapillare ist eine Druckmesseinrichtung, beispielsweise ein Drucksensor, vorhanden. Mindestens die erste Kapillare umfasst an ihrem Einlass eine Druckmesseinrichtung.

Die Kapillare ist im Einzelnen nicht beschränkt. Vorzugsweise werden ihre Länge und ihr Durchmesser so gewählt, dass die Durchströmzeit durch die Kapillare in etwa der Injektionszeit entspricht, die benötigt wird, um beispielsweise in einem Harz-Injektionsverfahren, eine bestimmte, vorgesehene Menge an Reaktionsharz zu fördern.

Die Viskositätsmessung kann durch Bestimmung der Druckdifferenz zwischen zwei Druckmesseinrichtungen nach dem Hagen-Poiseuill'schen Gesetz ermittelt werden:

$$V' = \frac{\pi \cdot r^4 \cdot \Delta p}{8 \cdot \eta \cdot l}$$

wobei V' der vordefinierte Volumenstrom in $m^3$/s durch die Kapillare, $\eta$ die Viskosität, r der Radius der Kapillare, $\Delta p$ die Druckdifferenz zwischen zwei Druckmesseinrichtungen und I die Länge der Kapillare ist.

Die erfindungsgemäße Vorrichtung hat zudem noch einen weiteren Vorteil: mit ihr ist es möglich über die ermittelte Viskosität die Verarbeitungszeit eines Reaktionsharzes zu bestimmen. Die Verarbeitungszeit ist dabei diejenige Zeit, in der das Reaktionsgemisch noch ausreichend fließfähig ist und bevor das Harz durch Reaktion mit dem Härter derart vernetzt ist, dass ein Fördern mit der gewünschten Geschwindigkeit nicht mehr möglich ist. Die Bestimmung der Verarbeitungszeit ist beispielsweise für Reaktionsharze, die in Injektionsvorrichtungen zur Herstellung von Faserverbundbauteilen und dergleichen verwendet werden, wichtig. Ist die Verarbeitungszeit bekannt, kann die Dosierung entsprechend eingestellt werden, so dass keine Fehlstellen oder Inhomogenitäten im gehärteten Harz auftreten.

Die erfindungsgemäße Vorrichtung ist damit vielseitig zur Viskositätsbestimmung von Reaktionsharzen einsetzbar und erlaubt eine schnelle, präzise und kostengünstige Ermittlung der Viskosität. Durch das einzigartige Dosiersystem besteht die Möglichkeit zur Vermengung mehrerer Substanzen, sowie auch zum Spülen der Vorrichtung mittels eines geeigneten Lösungsmittels nach Durchführung der Viskositätsmessung. Dadurch kann die Vorrichtung ohne Austausch einer ihrer Komponenten wiederverwendet werden. Der Aufbau der erfindungsgemäßen Vorrichtung ist kompakt und eignet sich somit auch als mobile Messvorrichtung.

Die Unteransprüche beinhalten vorteilhafte Weiterbildungen und Ausgestaltungen der Erfindung.

Gemäß einer vorteilhaften Weiterbildung umfasst die Dosiereinheit eine Pumpe und insbesondere eine Doppelkolbenpumpe. Hierdurch können Substanzen ohne Verwirbelungen dosiert werden, was Messfehlern vorbeugt.

Ebenfalls erfindungsgemäß wird auch ein Verfahren nach Anspruch 3 zur Herstellung der Vorrichtung nach Anspruch 1 zur Viskositätsbestimmung von Reaktionsharzen beschrieben.

Hierbei sei ausgeführt, dass sich das erfindungsgemäße Verfahren zur Herstellung der oben dargelegten erfindungsgemäßen Vorrichtung eignet. Das Verfahren umfasst die Schritte i) Bereitstellen einer HPLC (high-performance-liquid-chromatography)-Anlage, umfassend eine Probenaufbewahrungseinheit mit mindestens zwei Vorratsbehältnissen und jeweils einer Dosiereinheit, mindestens eine Mischvorrichtung, eine Hochdruckpumpe, eine Temperiereinheit und mindestens eine Trennsäule und ii) Ersetzen der Trennsäule durch vier oder fünf Kapillaren mit einem Einlass und einem Auslass, wobei mindestens an jedem Auslass einer Kapillare sowie am Einlass der ersten Kapillare eine Druckmesseinrichtung angeordnet wird.

Als Ausgangsbasis kann demnach eine herkömmliche HPLC-Anlage verwendet werden. Lediglich die Trennsäule wird durch eine Kapillare ersetzt und Druckmesseinrichtungen werden, wo erforderlich, vorgesehen. Somit ist auf sehr einfache Weise unter Verwendung herkömmlicher Gerätschaften, die Herstellung einer effizient, schnell und kostengünstig arbeitenden Vorrichtung zur Viskositätsbestimmung von Reaktionsharzen herstellbar. Das Verfahren ist darüber hinaus ohne hohen Aufwand umsetzbar.

Ferner erfindungsgemäß wird auch ein Verfahren nach Anspruch 4 zur Viskositätsbestimmung von Reaktionsharzen beschrieben. Das Verfahren umfasst folgende Schritte: i) ein Dosieren und Fördern eines härtbaren Harzes aus einem ersten Vorratsbehältnis einer Probenaufbewahrungseinheit mittels einer ersten Dosiereinheit und ii) ein Dosieren und Fördern eines Härters aus einem zweiten Vorratsbehältnis der Probenaufbewahrungseinheit mittels einer zweiten Dosiereinheit. Das härtbare Harz und der Härter werden dabei so ausgewählt, dass zwischen den Substanzen eine Härtungsreaktion, in der Regel eine Vernetzungsreaktion, initiiert werden kann. Diese erfolgt nach dem Schritt iii), dem Mischen des härtbaren Harzes und des Härters in einer Mischvorrichtung. Anschließend wird in Schritt iv) ein Einstellen eines vordefinierten Volumenstroms der Mischung aus härtbarem Harz und Härter durch eine Hochdruckpumpe ausgeführt, v) die Mischung aus härtbarem Harz und Härter temperiert und vi) die Reaktionsmischung, also die Mischung aus härtbarem Harz und Härter durch vier oder fünf in Reihe geschaltete Kapillaren mit einem Einlass und einem Auslass gefördert, wobei mindestens an jedem Auslass einer Kapillare sowie am Einlass der ersten Kapillare eine Druckmesseinrichtung angeordnet ist. Sodann vii) bestimmt man eine Druckdifferenz, die sich zwischen zwei Druckmesseinrichtungen bei gegebenem Volumenstrom des Gemisches aus Härter und härtbarem Harz einstellt. Hieraus kann, wie vorstehend für die erfindungsgemäße Vorrichtung zur Viskositätsbestimmung von Reaktionsharzen beschrieben, gemäß dem Hagen-Poiseuill'schen Gesetz die Viskosität bestimmt werden. Das Verfahren ist zeitsparend umsetzbar und erlaubt eine präzise Bestimmung der Viskosität von Reaktionsharzen.

Bezüglich weiterer Details wird ergänzend Bezug genommen auf die vorstehenden Ausführungen zur erfindungsgemäßen Vorrichtung zur Viskositätsbestimmung von Reaktionsharzen.

Vorzugsweise wird nach Durchfluss der Mischung aus härtbarem Harz und Härter die Kapillare durch Dosieren und Fördern eines Lösungsmittels für das gehärtete Harz aus einem dritten Vorratsbehältnis durch die Kapillare von Rückständen befreit.

Weitere Einzelheiten, Merkmale und Vorteile der Erfindung ergeben sich aus der nachfolgenden Beschreibung und die Figur. Es zeigt:

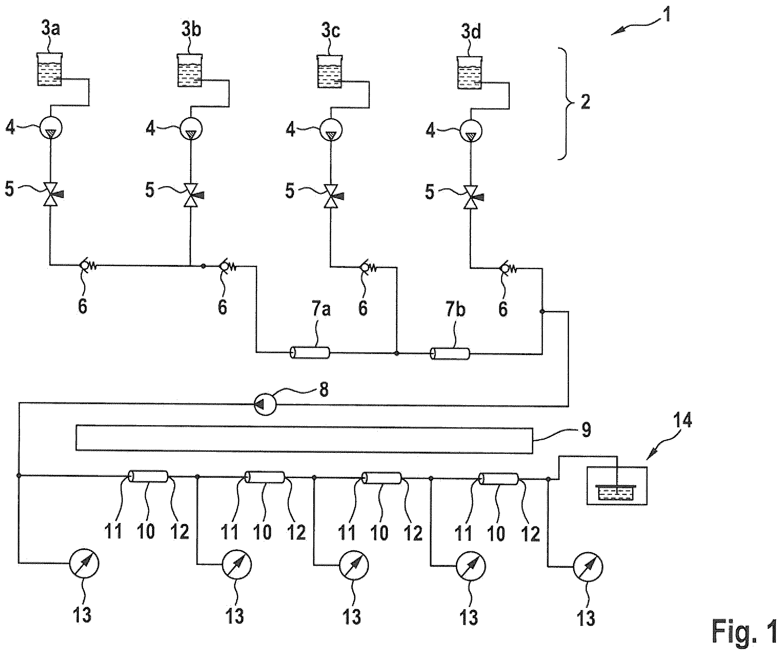

Figur 1    eine schematische Darstellung einer Vorrichtung zur Viskositätsbestimmung von Reaktionsharzen gemäß einer vorteilhaften Ausgestaltung der Erfindung.

Die vorliegende Erfindung wird anhand eines Ausführungsbeispiels im Detail erläutert. In Figur 1 sind hierzu nur die erfindungswesentlichen Aspekte der Erfindung dargestellt. Alle übrigen Aspekte sind der Übersichtlichkeit halber weggelassen.

Die erfindungsgemäße Vorrichtung 1 umfasst eine Probenaufbewahrungseinheit 2 mit vier Vorratsbehältnissen 3a, 3b, 3c, 3d. An die Vorratsbehältnisse 3a, 3b, 3c, 3d schließt sich jeweils eine Dosiereinheit 4 an, mit der eine entsprechende Menge einer bevorrateten Substanz dosiert und gefördert werden kann. Nadelventile 5 und Rückstromventile 6 verhindern ein Zurückfließen der Substanzen.

Die aus den Vorratsbehältnissen 3b und 3c sowie aus den Vorratsbehältnissen 3b/3c und 3d geförderten Substanzen werden in Mischvorrichtungen 7a bzw. 7b miteinander vermischt.

Die Probenaufbewahrungseinheit 2 und die Mischvorrichtungen 7a, 7b befinden sich im so genannten Niederdruckbereich der Vorrichtung 1. Dies bedeutet, dass hier im Wesentlichen keine Druckerhöhung stattgefunden hat, und sich die Substanzen und Gemische in der Regel auf Normaldruck befinden.

An den Niederdruckbereich schließt sich ein Hochdruckbereich an. Dieser wird nach Passieren einer Hochdruckpumpe 8 erreicht.

Die Vorrichtung enthält im Hochdruckbereich beispielhaft fünf Kapillaren 10. Jede Kapillare hat einen Einlass 11 und einen Auslass 12. An jedem Auslass 12 jeder Kapillare 10 ist eine Druckmesseinrichtung 13 angeordnet. Ebenso ist auch am Einlass 11 der ersten Kapillare 10 eine Druckmesseinrichtung 13 angeordnet. Somit ist jeweils am Einlass 11 und am Auslass 12 einer jeden Kapillare 10 eine Druckmesseinrichtung 13 vorhanden. Damit kann eine Druckdifferenz zwischen dem Einlass 11 einer betrachteten Kapillare 10 und dem Auslass 12 dieser Kapillare 10 gemessen werden. Ebenso ist es möglich eine Druckdifferenz über mehrere Kapillaren 10 hinweg zu bestimmen.

Die Vorrichtung umfasst ferner eine Temperiereinheit 9, mit der alle Kapillaren 10 auf eine gewünschte Temperatur gebracht werden können.

Den Kapillaren 10 nachgeschaltet ist ein Auffangbehältnis 14, in dem durch die Kapillaren geförderte Substanzen für die anschließende Entsorgung aufgefangen werden.

Anhand des folgenden Beispiels soll die Funktionsweise der Vorrichtung 1 erläutert werden:
Die Vorratsbehältnisse 3a-3d sind wie folgt befüllt:

| | |
|---|---|
| Vorratsbehältnis 3a: | Lösungsmittel für ein Reaktionsharz |
| Vorratsbehältnis 3b: | Härter |
| Vorratsbehältnis 3c: | Trennmittel |
| Vorratsbehältnis 3d: | härtbares Harz |

Aus dem Vorratsbehältnis 3b wird über die sich anschließende Dosiereinheit 4 eine vordefinierte Menge an Härter dosiert und gefördert. Aus dem Vorratsbehältnis 3d wird über die sich anschließende Dosiereinheit 4 eine vordefinierte Menge an härtbarem Harz dosiert und gefördert. Das härtbare Harz und der Härter werden in Mischvorrichtung 7b miteinander vermischt und in der Leitung zur Hochdruckpumpe 8 gefördert.

Durch die Hochdruckpumpe 8 wird ein vordefinierter Volumenstrom der Mischung aus härtbarem Harz und Härter eingestellt.

Nach Passieren der Hochdruckpumpe 8 wird das Gemisch aus Härter und härtbarem Harz bei konstanter Temperatur, die durch die Temperiereinheit 9 eingestellt und gehalten wird, durch die Kapillaren 10 gefördert. Durch die Druckmessvorrichtungen 13 kann eine Druckdifferenz an beliebiger Stelle zwischen den Kapillaren 10 ermittelt werden.

Bestimmt man nun eine Druckdifferenz, die sich zwischen zwei Druckmesseinrichtungen 13 bei gegebenem Volumenstrom des Gemisches aus Härter und härtbarem Harz einstellt, so kann hieraus die Viskosität des Reaktionsharzes, also des Gemisches aus Härter und härtbarem Harz, über das Hagen-Poiseuill'sche Gesetz bestimmt werden.

Ebenso kann durch Versuche und einen Vergleich der ermittelten Parameter die Zeit ermittelt werden, die ein Reaktionsharz noch ausreichend fließfähig ist, bevor es vollständig durchgehärtet ist. Diese Erkenntnisse sind hilfreich für die Prozessführung in einem Injektionsverfahren des betrachteten Reaktionsharzes, beispielsweise in einem RTM-Verfahren.

Nach beendeter Viskositätsmessung kann beispielsweise aus dem Vorratsbehältnis 3a ein Lösungsmittel für das Reaktionsharz gefördert und durch die Kapillaren 10 gepumpt werden. Dies verhindert ein Verblocken des Kapillaren 10 mit Harz und ermöglicht eine wiederholte Verwendung der Kapillaren 10.

Ebenfalls ist es möglich aus dem Vorratsbehältnis 3c ein Trennmittel zuzuführen.

Die vorhergehende Beschreibung der vorliegenden Erfindung dient nur zu illustrativen Zwecken und nicht zum Zwecke der Beschränkung der Erfindung. Im Rahmen der Erfindung sind verschiedene Änderungen und Modifikationen möglich, ohne den Umfang der Erfindung sowie ihrer Äquivalente zu verlassen.

**Bezugszeichenliste:**

| 1 | Vorrichtung zur Viskositätsbestimmung von Reaktionsharzen |
|---|---|
| 2 | Probenaufbewahrungseinheit |
| 3a | Vorratsbehältnis |
| 3b | Vorratsbehältnis |
| 3c | Vorratsbehältnis |
| 3d | Vorratsbehältnis |
| 4 | Dosiereinheit |
| 5 | Nadelventil |
| 6 | Rückstromventil |
| 7a | Mischvorrichtung |
| 7b | Mischvorrichtung |
| 8 | Hochdruckpumpe |
| 9 | Temperiereinheit |
| 10 | Kapillare |
| 11 | Einlass der Kapillare |
| 12 | Auslass der Kapillare |
| 13 | Druckmesseinrichtung |
| 14 | Auffangbehältnis |

**Patentansprüche**

1. Vorrichtung zur Viskositätsbestimmung von Reaktionsharzen umfassend:

   - eine Probenaufbewahrungseinheit (2) mit mindestens zwei Vorratsbehältnissen (3a, 3b, 3c, 3d) und jeweils einer Dosiereinheit (4), wobei ein erstes Vorratsbehältnis (3a) zum Aufbewahren eines härtbaren Harzes vorgesehen ist und ein zweites Vorratsbehältnis (3b) zum Aufbewahren eines Härters vorgesehen ist,
   - mindestens eine Mischvorrichtung (7a, 7b) zum Mischen des härtbaren Harzes und des Härters,
   - eine Hochdruckpumpe (8) zum Bringen der Mischung aus härtbarem Harz und Härter auf einen vorbestimmten Druck,
   - eine Temperiereinheit (9) zum Temperieren der Mischung aus härtbarem Harz und Härter und
   - vier oder fünf in Reihe geschaltete Kapillaren (10) mit je einem Einlass (11) und je einem Auslass (12), wobei mindestens an jedem Auslass einer Kapillare (12) sowie am Einlass der ersten Kapillare (11) eine Druckmesseinrichtung (13) angeordnet ist, zur Bestimmung der Viskosität der Mischung aus härtbarem Harz und Härter.

2. Vorrichtung nach Anspruch 1, wobei die Dosiereinheit (4) eine Pumpe, insbesondere eine Doppelkolbenpumpe, umfasst.

3. Verfahren zur Herstellung der Vorrichtung zur Viskositätsbestimmung von Reaktionsharzen (1) nach Anspruch 1 oder 2, umfassend die Schritte:

   - Bereitstellen einer HPLC-Anlage, umfassend eine Probenaufbewahrungseinheit (2) mit mindestens zwei Vorratsbehältnissen (3a, 3b, 3c, 3d) und jeweils einer Dosiereinheit (4), mindestens eine Mischvorrichtung (7a, 7b), eine Hochdruckpumpe (8), eine Temperiereinheit (9) und mindestens eine Trennsäule und
   - Ersetzen der Trennsäule durch vier oder fünf in Reihe geschaltete Kapillaren (10) mit einem Einlass (11) und einem Auslass (12), wobei mindestens an jedem Auslass einer Kapillare (12) sowie am Einlass der ersten Kapillare (11) eine Druckmesseinrichtung (13) angeordnet wird.

4. Verfahren zur Viskositätsbestimmung von Reaktionsharzen, umfassend die Schritte:

- Dosieren und Fördern eines härtbaren Harzes aus einem ersten Vorratsbehältnis (3d) einer Probenaufbewahrungseinheit (2) mittels einer ersten Dosiereinheit (4),
- Dosieren und Fördern eines Härters aus einem zweiten Vorratsbehältnis (3b) der Probenaufbewahrungseinheit (2) mittels einer zweiten Dosiereinheit (4),
- Mischen des härtbaren Harzes und des Härters in einer Mischvorrichtung (7b),
- Einstellen eines vordefinierten Volumenstroms der Mischung aus härtbarem Harz und Härter durch eine Hochdruckpumpe (8),
- Temperieren der Mischung aus härtbarem Harz und Härter,
- Fördern der Mischung aus härtbarem Harz und Härter durch vier oder fünf in Reihe geschaltete Kapillaren (10) mit einem Einlass (11) und einem Auslass (12), wobei mindestens an jedem Auslass einer Kapillare (12) sowie am Einlass der ersten Kapillare (11) eine Druckmesseinrichtung (13) angeordnet ist und
- Bestimmen einer Druckdifferenz, die sich zwischen zwei Druckmesseinrichtungen 13 bei gegebenem Volumenstrom des Gemisches aus Härter und härtbarem Harz einstellt.

5. Verfahren nach Anspruch 4, **dadurch gekennzeichnet, dass** nach Durchfluss der Mischung aus härtbarem Harz und Härter die Kapillaren (10) durch Dosieren und Fördern eines Lösungsmittels für das gehärtete Harz aus einem dritten Vorratsbehältnis (3a) durch die Kapillaren (10) von Rückständen befreit werden.

**Claims**

1. Apparatus for determining the viscosity of reactive resins, comprising:

- a sample storage unit (2) having at least two storage containers (3a, 3b, 3c, 3d) and in each case one dosing unit (4), wherein a first storage container (3a) is provided for storing a curable resin, and a second storage container (3b) is provided for storing a curing agent,
- at least one mixing apparatus (7a, 7b) for mixing the curable resin and the curing agent,
- a high-pressure pump (8) for bringing the mixture of curable resin and curing agent to a predetermined pressure,
- a temperature-control unit (9) for controlling the temperature of the mixture of curable resin and curing agent, and
- four or five capillaries (10) which are connected in series and have in each case one inlet (11) and in each case one outlet (12), wherein, for the purpose of determining the viscosity of the mixture of curable resin and curing agent, a pressure measurement device (13) is arranged at least at each outlet of a capillary (12) and at the inlet of the first capillary (11).

2. Apparatus according to Claim 1, wherein the dosing unit (4) comprises a pump, in particular a double-piston pump.

3. Method for producing the apparatus for determining the viscosity of reactive resins (1) according to Claim 1 or 2, comprising the steps of:

- providing an HPLC system, comprising a sample storage unit (2) having at least two storage containers (3a, 3b, 3c, 3d) and in each case one dosing unit (4), at least one mixing apparatus (7a, 7b), a high-pressure pump (8), a temperature-control unit (9) and at least one separating column, and
- replacing the separating column by four or five capillaries (10) which are connected in series and have an inlet (11) and an outlet (12), wherein a pressure measurement device (13) is arranged at least at each outlet of a capillary (12) and at the inlet of the first capillary (11).

4. Method for determining the viscosity of reactive resins, comprising the steps of:

- dosing and delivering a curable resin from a first storage container (3d) of a sample storage unit (2) by means of a first dosing unit (4),
- dosing and delivering a curing agent from a second storage container (3b) of the sample storage unit (2) by means of a second dosing unit (4),
- mixing the curable resin and the curing agent in a mixing apparatus (7b),

- setting a predefined volumetric flow rate of the mixture of curable resin and curing agent by way of a high-pressure pump (8),
- controlling the temperature of the mixture of curable resin and curing agent,
- delivering the mixture of curable resin and curing agent through four or five capillaries (10) which are connected in series and have an inlet (11) and an outlet (12), wherein a pressure measurement device (13) is arranged at least at each outlet of a capillary (12) and at the inlet of the first capillary (11), and
- determining a pressure difference which is established between two pressure measurement devices (13) at the given volumetric flow rate of the mixture of curing agent and curable resin.

5. Method according to Claim 4, **characterized in that**, after throughflow of the mixture of curable resin and curing agent, the capillaries (10) are freed of residues by dosing and delivering a solvent for the cured resin from a third storage container (3a) through the capillaries (10).

**Revendications**

1. Dispositif de détermination de la viscosité de résines réactives, ledit dispositif comprenant :

- une unité de stockage d'échantillons (2) pourvue d'au moins deux réservoirs (3a, 3b, 3c, 3d) et, pour chacun de ceux-ci, d'une unité de dosage (4), un premier réservoir (3a) étant prévu pour stocker une résine durcissable et un deuxième réservoir (3b) étant prévu pour stocker un durcisseur,
- au moins un dispositif de mélange (7a, 7b) destiné à mélanger la résine durcissable et le durcisseur,
- une pompe à haute pression (8) destinée à amener le mélange de résine durcissable et de durcisseur à une pression prédéterminée,
- une unité de régulation de température (9) destinée à réguler la température du mélange de résine durcissable et de durcisseur et
- quatre ou cinq capillaires (10) montés en série, chacun d'eux étant pourvu d'une entrée (11) et d'une sortie (12), un dispositif de mesure de pression (13) étant disposé au moins au niveau de chaque sortie d'un capillaire (12) et à l'entrée du premier capillaire (11) pour déterminer la viscosité du mélange de résine durcissable et de durcisseur.

2. Dispositif selon la revendication 1, l'unité de dosage (4) comprenant une pompe, notamment une pompe à double piston.

3. Procédé de fabrication du dispositif de détermination de la viscosité de résines réactives (1) selon la revendication 1 ou 2, ledit procédé comprenant les étapes suivantes :

- fournir un système HPLC comprenant une unité de stockage d'échantillons (2), pourvue d'au moins deux réservoirs (3a, 3b, 3c, 3d) et, pour chacun de ceux-ci, d'une unité de dosage (4), au moins un dispositif de mélange (7a, 7b), une pompe à haute pression (8), une unité de régulation de température (9) et au moins une colonne de séparation et
- remplacer la colonne de séparation par quatre ou cinq capillaires (10) montés en série et pourvus d'une entrée (11) et d'une sortie (12), un dispositif de mesure de pression (13) étant disposé au moins au niveau de chaque sortie d'un capillaire (12) et de l'entrée du premier capillaire (11).

4. Procédé de détermination de la viscosité de résines réactives, ledit procédé comprenant les étapes suivantes :

- doser et acheminer une résine durcissable depuis un premier réservoir (3d) d'une unité de stockage d'échantillons (2) au moyen d'une première unité de dosage (4),
- doser et acheminer un durcisseur depuis un deuxième réservoir (3b) de l'unité de stockage d'échantillons (2) au moyen d'une deuxième unité de dosage (4),
- mélanger la résine durcissable et le durcisseur dans un dispositif de mélange (7b),
- régler un débit volumique prédéfini du mélange de résine durcissable et de durcisseur au moyen d'une pompe à haute pression (8),
- réguler la température du mélange de résine durcissable et de durcisseur,
- acheminer le mélange de résine durcissable et de durcisseur à travers quatre ou cinq capillaires (10) montés en série et pourvus d'une entrée (11) et d'une sortie (12), un dispositif de mesure de pression (13) étant disposé au niveau de chaque sortie d'un capillaire (12) et à l'entrée du premier capillaire (11)

- déterminer une différence de pression qui est réglée entre deux dispositifs de mesure de pression (13) pour un débit volumique donné du mélange de durcisseur et de résine durcissable.

5. Procédé selon la revendication 4, **caractérisé en ce qu'**après écoulement du mélange de résine durcissable et de durcisseur, des résidus sont éliminés des capillaires (10) par dosage et acheminement d'un solvant, destiné à la résine durcie, depuis un troisième réservoir (3a) à travers les capillaires (10) .

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5076096 A